# EUROPEAN PATENT APPLICATION

(11) **EP 1 291 430 A1**
(43) Date of publication of application: **12.03.2003**
(21) Application number: 01936941.2
(22) Date of filing: 11.06.2001
(51) Int. Cl.: C12N 15/54, C12N 9/12, C12N 1/21, C12Q 1/48, C07K 16/40, A61K 31/7125, A61K 48/00, A61P 3/04, A61P 3/10, A61P 7/00, A61P 25/00, A61P 29/00, A61P 31/18, A61P 35/00

(54) **CERAMIDE KINASE AND DNA ENCODING THE SAME**

(30) Priority: 14.06.2000 JP 2000178039
(71) Applicant: Sankyo Company, Limited, Tokyo 103-8426 (JP)
(72) Inventor: SUGIURA, Masako, c/o SANKYO COMPANY, LIMITED, Tokyo 140-8710 (JP); KONO, Keita, c/o SANKYO COMPANY, LIMITED, Tokyo 140-8710 (JP); KOHAMA, Takafumi, c/o SANKYO COMPANY, LIMITED, Tokyo 140-8710 (JP)
(74) Representative: Gibson, Christian John Robert
(86) International application number: JP0104889
(87) International publication number: WO01096575

(57) **Abstract**

The present invention provides a novel protein having ceramide kinase activity which can be a target for a prophylactic or therapeutic medicament against neuronal disease, inflammation, HIV infection, type 2 diabetes mellitus, obesity, septicemia, arteriosclerosis and cancer.

Specifically, a protein which comprises an amino acid sequence shown in the amino acid numbers 1-537 of SEQ ID No. 2 of the Sequencing Listing, a DNA which encodes the protein, a recombinant vector comprising the DNA, a host cell transformed with the recombinant vector, a method for producing the protein, and utilities of the protein are provided.

By using the method of the present invention, a novel compound is provided having a specifically activating or inhibiting activity to ceramide kinase and useful as a medicament for treating neuronal disorder, an anti-inflammatory medicament, a medicament for treating HIV infection, an anti-type 2 diabetes mellitus medicament, an anti-obesity medicament, an anti-septicemia medicament, an anti-arteriosclerosis medicament, and an anticancer medicament.

## Description

### [Field of the Invention]

The present invention relates to a novel protein having ceramide kinase activity which may be a target of a medicament preventing or treating neuronal disease, inflammation, HIV infection, type 2 diabetes mellitus, obesity, septicemia, arteriosclerosis and cancer, DNA which encodes the protein, a recombinant DNA vector comprising the DNA, a host transformed with the recombinant DNA vector, a method for producing the ceramide kinase, and use of the protein.

### [Background of the Invention]

Sphingolipids have been conventionally considered to be one of the major components of cell membrane as glycerophospholipids and cholesterol. It has been known that glycerophospholipids not only maintain cell membrane structure but also let the cells produce a lot of physiologically active substances as metabolites thereof. However, a physiological activity of sphingolipid metabolites has been hardly known until recently. In these years, physiological activities of some sphingolipid metabolites such as induction of apoptosis and cell proliferation-stimulating activity, have come to be known, and the possibility that enzymes metabolizing sphingolipid closely participate in physiological reactions or various diseases has been suggested. Especially, ceramide attracts attention as a regulator which controls a lot of cell mechanisms (See Hannun, Y.A.and Obeid, L.M. (1995) TIBS 20, 73-77).

For example, it is known that ceramide functions as a second messenger of inflammatory cytokines, such as TNF-α and IL-1β, and activates arachidonic acid pathways such as phospholipase A₂ and the like (See Spiegel, S. et al.(1996) Curr. Opini. Cell Biol. 8, 159-167 and Mathias S. et al., (1993) Science 259, 519-522). That is, ceramide can be considered to be an exacerbating factor in various inflammatory disorders. Moreover, it has also been reported that ceramide functions as an exacerbating factor in reduction of CD4⁺ T cell accompanied by apoptosis and HIV infection of brain cells in patients infected with HIV (See Coline, M. G et al. (1997) Proc. Assoc. Am. Physicians 109, 146-153 and Wilt, S. et al. (1995) Ann. Neurol. 37, 381-394). Furthermore, it is known that TNF-α causes insulin resistance in type 2 diabetes mellitus and obesity, and it is thought that ceramide functions as an exacerbating factor also on the downstream thereof (See Begum, N. et al. (1996) Eur. J. Biochem. 238, 214-220 and Hotamisligil, G S. et al. (1996) Science 271, 665 to 668). Moreover, it has also been reported that ceramide functions as a second messenger in septicemia caused by lipopolysaccharide (LPS) or the like as a trigger (see Beutler, B. and Kruys, V. (1995) J. Cardiovasc. Pharmacol. 25, S1-S8). Furthermore, it has also been reported that increase of ceramide accompanied by the activation of sphingomyelinase functions as an exacerbating factor in the aggregating reaction of LDL which is to be a trigger of arteriosclerosis lesions (See Schissel, S. L. et al. (1996) J. Clin. Invest. 98, 1455-1464).

On the contrary, it is known that ceramide promotes apoptosis of cancer cells in radiotherapy and chemotherapy for cancer treatments (See Michael, J. M. et al. (1997) Cancer Res. 57, 3600-3605 and Bose, R. et al.(1995) Cell 82, 405-414 and Jaffrezou, J. P. et al. (1996) EMBO J. 15, 2417-2424).

On the other hand, some physiological activities are known also about ceramide-1-phosphate, a metabolite of ceramide and ceramide kinase, a ceramide-1-phosphate-producing enzyme. For example, ceramide-1-phosphate produced through the activation of ceramide kinase responding to calcium stimulus regulates the release of neuronal transmitters from brain synapses (Bajjalieh, S. M. et al.(1989) J. Biol. Chem. 264, 14354-14360 and Shinghal, R. et al. (1993) J. Neurochem. 61, 2279 -2285). Therefore, it may be possible to treat various neuronal disorders including Alzheimer's disease by regulating the activity of ceramide kinase with a medicament or the like. Moreover, it is considered that ceramide-1-phosphate has an activity to block the various mechanisms of ceramides mentioned above (See Dressler, K. A. et al. (1990) J. Biol. Chem. 265, 14917-14921). That is, it is thought that the disorders to which ceramide acts as an exacerbating factor, including various inflammatory disorders such as chronic arthritis, HIV infection, and type 2 diabetes mellitus caused by insulin resistance as a trigger, obesity, septicemia and arteriosclerosis, can be suppressed by using ceramide-1-phosphate. Therefore, it can be a method for treating various inflammatory disorders, HIV infection, type 2 diabetes, obesity, septicemia, arteriosclerosis and the like to activate ceramide kinase with a medicament or the like. On the contrary, in the radiotherapy and chemotherapy of cancer, the amount of ceramide can be maintained and the therapeutic efficacy can be improved by inhibiting ceramide kinase activity.

Moreover, quantification of ceramide is considered to be important for analyzing the condition of various diseases for the above-mentioned reasons. By using ceramide kinase, it is possible to provide a simple, specific and highly sensitive method for quantifying the ceramide content in a cell or a tissue. Although a lot of methods such as mass spectrometry and HPLC are known as methods for quantifying ceramide, there are problems in sensitivity or simplicity. While a method using diacylglycerol kinase is also known (See Van Veldhoven, P. P. et al. (1995) Biochem. Mol. Biol. Int. 36, 21-30), said enzyme is an enzyme which essentially phosphorylates diacylglycerol, and is not sufficient in its specificity and specific activity for ceramide. Therefore, a specific and sensitive method for quantifying or detecting ceramide can be established by using ceramide kinase.

However, there is no report wherein the ceramide kinase which is an enzyme for producing ceramide-1-phosphate is purified or cloned, and many of its details have remained unknown. Then, it has been desired to clone the ceramide kinase gene and express the protein encoded thereby, so that it may be preferably used for screening a therapeutic or prophylactic medicament for the various above-mentioned disorders.

In addition, an expresseed sequence tag (hereinafter referred to as "EST") corresponding to a part of the DNA which encodes the protein of the present invention is known, when a homology search is performed by using the dbEST database of NCBI (National Center for Biotechnology Information, U.S.A.) based on the sequence of known mouse sphingosine kinase. However, there is no specific suggestion regarding the function of the protein of the present invention in said EST data.

That is, the purpose of the present invention is to clone the gene encoding a novel ceramide kinase which exists in a human, to provide a novel method for screening a candidate substance as a medicament for treating neuronal disorders, an anti-inflammatory medicament, a medicament for treating the HIV infection, an anti-type 2 diabetes mellitus medicament, an anti-obesity medicament, a medicament for treating septicemia, an anti-arteriosclerosis medicament and an anticancer medicament thereby, and to provide a method for analyzing various conditions of the disorders. Such a search for candidate substances as a novel type of medicament is performed by screening substances which activate or inhibit the ceramide kinase activity of the protein of the present invention. The analysis of various conditions of the disorders can be performed by labeling ceramides in various samples with the protein of the present invention.

### [Disclosure of the invention]

The inventors of the present invention cloned cDNA which encodes a novel protein which is partially homologous to mouse sphingosine kinase, and determined successfully the primary structure of the full length novel protein. Furthermore, they successfully allowed the novel protein to be expressed, and confirmed that the novel protein has ceramide kinase activity. Thereby, the present invention was completed.

First, the present invention relates to a protein described in any one of the following (i) to (iii):
(i) a protein which comprises an amino acid sequence shown in the amino acid numbers 1-537 of SEQ ID No. 2 of the Sequencing Listing;
(ii) a protein which comprises an amino acid sequence wherein one or more than one amino acids are added, deleted and/or replaced in the amino acid sequence shown in the amino acid numbers 1-537 of SEQ ID No. 2 of the Sequencing Listing, and has ceramide kinase activity;
(iii) a protein encoded by DNA which is inserted in the plasmid carried by a transformed Escherichia coli E.coli pCR3.1-CERK1 SANK 70300 (FERM BP-7184).

Secondly, the present invention relates to DNA which encodes the above-mentioned protein. Such DNA of the present invention is preferably DNA comprising a nucleotide sequence shown in the nucleotide numbers 124-1734 of SEQ ID No. 1 of the Sequencing Listing; DNA which hybridizes with the DNA which comprises a nucleotide sequence shown in the nucleotide numbers 124-1734 of SEQ ID No. 1 of the Sequencing Listing under stringent conditions, and encodes a protein having ceramide kinase activity; or DNA inserted in the plasmid which is carried by transformed Escherichia coli E.coli pCR3.1-CERK1 SANK 70300 (FERM BP-7184). However, the present invention is not limited thereto, but includes all the DNAs having the nucleotide sequence which encodes the above-mentioned protein.

Thirdly, the present invention relates to a recombinant DNA vector comprising the above-mentioned DNA. While the preferable recombinant DNA vector of the present invention is pCR3.1-CERK1, a recombinant DNA vector, which is carried by transformed Escherichia coli E.coli pCR3.1-CERK1 SANK 70300 (FERM BP-7184), or a recombinant DNA vector which is an expression vector, the present invention is not limited thereto.

Fourthly, the present invention relates to a host cell transformed with the above-mentioned recombinant DNA vector. Such a host cell of the present invention is preferably transformed Escherichia coli E.coli pCR3.1-CERK1 SANK 70300 (FERM BP-7184), or a host cell transformed with the recombinant DNA vector which is an expression vector of the present invention, but the present invention is not limited thereto.

Fifthly, the present invention relates to a method for producing the above-mentioned protein. One example thereof is one based on a genetic engineering procedure wherein the host cell transformed with the above-mentioned expression vector is cultured under conditions wherein the protein having ceramide kinase activity can be produced, and subsequently a protein having ceramide kinase activity is collected from the culture.

Another example of the method for producing the protein of the present invention is based on a biochemical procedure which comprises the following steps (i) to (iii):
(i) an extract of a material containing a protein according to any one of i) to iii) dissolved in a solvent which does not contain calmodulin is allowed to adsorb on affinity-chromatography resin having calmodulin as a substituent;
(ii) the resin of (i) is washed with the solvent which does not contain calmodulin;
(iii) a protein having ceramide kinase activity is eluted with a solvent containing ethylene glycol bis (β-aminoethyl ether)- N,N,N',N'-tetraacetic acid (EGTA) or ethylenediaminetetraacetic acid (EDTA) from the resin washed in the step (ii).

Sixthly, the present invention relates to a method for testing the ceramide kinase-activating or inhibiting activity of a compound or a composition wherein the above-mentioned protein, a substrate which can be specifically phosphorylated by the protein, and the compound or the composition sample are allowed to coexist, and then the amount of the substrate phosphorylated by the protein is measured, and the resulting amount is compared with that without addition of the compound or the composition sample thereto.

Seventhly, the present invention relates to a method for detecting or quantifying ceramide in a sample wherein the above-mentioned protein, the sample to be tested, and labeled adenosine 5'-triphosphate (hereinafter referred to as "ATP") are allowed to coexist and then the labeled ceramide is detected or quantified.

Eighthly, the present invention relates to an antibody which specifically binds to the above-mentioned protein which is a novel protein.

That is, the present invention provides a novel protein, DNA which encodes the protein, a recombinant vector comprising the DNA, a host cell transformed with the vector, a method for producing the protein, use of the protein, and an antibody specifically binding to the protein.

In the present invention, the word "ceramide kinase activity" means an activity to phosphorylate the hydroxyl group at the 1-position of various ceramides such as the N-acetyl form of D-erythro-sphingosine (hereinafter referred to as "C₂-ceramide"), the N-hexanoyl form of D-erythro-sphingosine (hereinafter referred to as "C₆-ceramide"), the N-octanoyl form of D-erythro-sphingosine (hereinafter referred to as "C₈-ceramide"), the N-palmitoyl form of D-erythro-sphingosine, (hereinafter referred to as "C₁₆-ceramide"), and ceramide derived from bovine fetal brain, and various optical isomers thereof, resulting in the production of ceramide-1-phosphate. Namely, it means an activity for catalyzing a reaction shown by the following formula: 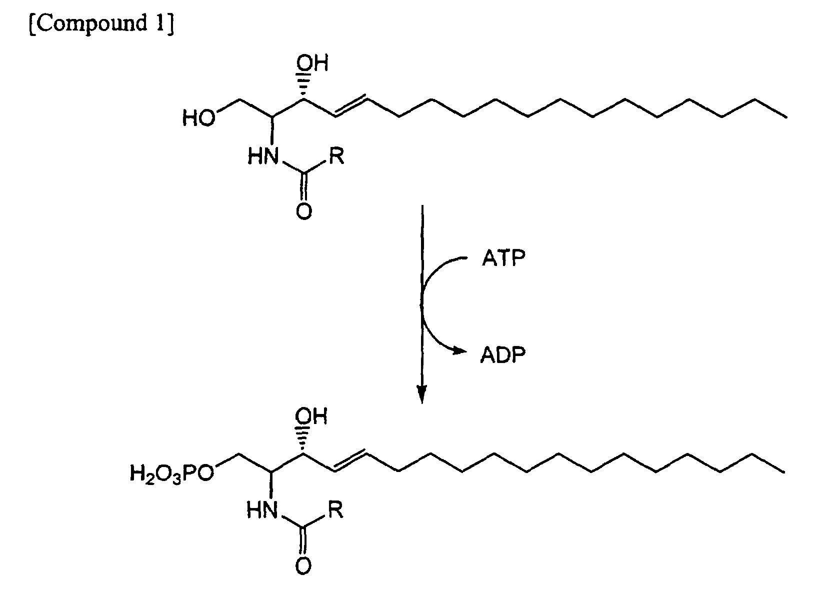 wherein R represents CH₃ (in the case of C₂-ceramide), C₅H₁₁ (in the case of C₆-ceramide), C₇H₁₅ (in the case of C₈-ceramide), C₁₅H₃₁ (in the case of C₁₆-ceramide), C₁₇H₃₅ (in the case of C₁₈-ceramide), or the like. Moreover, the preferable substrates which can be phosphorylated by the ceramide kinase activity possessed by the protein of the present invention are various ceramides such as C₂-ceramide, C₆-ceramide, C₈-ceramide, C₁₆-ceramide, and a ceramide derived from bovine fetal brain (mainly containing C₁₈-ceramide) and the various optical isomers thereof, and more preferably C₆-ceramides, but not limited thereto.

DNA which encodes the protein of the present invention can be obtained by a method well known to the skilled person in the art of the present invention, for example, by preparing mRNA from the cultured cells which expresses the protein, then synthesizing cDNA using it as a template, and screening cDNA which encodes the protein of the present invention by a well-known method, or the like.

More specifically, for instance, part or all of the DNA which encodes the protein of the present invention can be obtained by preparing a probe based on the full sequence or a partial sequence of cDNA of mouse sphingosine kinase, and analyzing the sequence of the clones obtained from a cDNA library derived from mammals by performing a colony-hybridization method under the conditions of low stringency, and selecting the clone having a sequence highly homologous to the nucleotide sequence shown in the nucleotide numbers 124-1734 of SEQ ID No. 1 of the Sequencing Listing. Moreover, a part of the DNA which encodes the protein of the present invention can be obtained also by preparing primers based on the known sequence of mouse sphingosine kinase (preferably primers are prepared based on the sequence of the conserved region in phylogeny), and performing a polymerase chain reaction (hereinafter referred to as "PCR", see Saiki R. K. et al.(1988) Science 239, 487 to 491) using the cDNA library derived from mammals as a template, or performing reverse-transcriptase polymerase chain reaction (hereinafter referred to as "RT-PCR") using mRNA derived from mammals as a template, and analyzing the resultant sequence the product has and selecting the clone having a sequence which is highly homologous to the nucleotide sequences shown in the nucleotide numbers 124-1734 of SEQ ID No. 1 of the Sequencing Listing.

Furthermore, full length cDNA which encodes the protein of the present invention can be obtained, for example, by preparing a probe based on the partial sequence obtained as mentioned above, and analyzing the sequence of the clone obtained by conducting colony-hybridization under stringent conditions over the cDNA library derived from mammals, or by preparing a probe based on the partial sequence, and performing PCR using the cDNA library derived from mammals as a template or RT-PCR using mRNA derived from mammals as a template, and then analyzing the sequence of the product, and furthermore performing the rapid amplification of cDNA ends (hereinafeter referred to as "RACE", See Jikken-Igaku, (1994), 12 (6), 35-38) if necessary, using a primer based on a partial sequence and mRNA derived from mammals or using commercially available kit and a commercially available cDNA library.

An animal cell used as a source of the mRNA is preferably human embryo kidney cell HEK293 (ATCC CRL-1573), but cells or tissues derived from mammals, or the other cultured-cell strains (including those of human origin) can also be used.

Extraction of mRNA can be performed by the guanidinium - cesium chloride ultracentrifugation method, the guanidinium thiocyanate-hot phenol method, the guanidinium thiocyanate - HCl method, the acidic guanidinium thiocyanate - phenol-chloroform method, but a commercially available mRNA separation kit can also be used.

Since it is known that many mRNAs existing in the cytoplasm of a eukaryotic cell have a poly (A) sequence at the 3'-end thereof, mRNA can be adsorbed on a biotinized oligo (dT) probe on the basis of this property, and purified by catching mRNA to the paramagnetic grain wherein streptoavidin is fixed by using the binding between biotin and streptoavidin, and eluting the mRNA after a washing operation. Moreover, mRNA can also be further fractionated by the sucrose density gradient centrifugation method or the like.

In order to confirm that the mRNA obtained as described above encodes a protein having a ceramide kinase activity, mRNA can be translated into protein, and the enzyme activity thereof can be examined. Alternatively, a method for identifying the protein with an antibody specific to the protein can be used. For example, mRNA is injected into an oocyte of a Xenopus (Xenopus laevis), and can be translated (Gardon j. B. et al. (1972) Nature 233, 177-182). Alternatively, non-cell translation systems, such as a rabbit reticulocyte system and a wheat-germ system, can also be used (Schleif, R. F. and Wensink, P. C. (1981): "Practical Methods in Molecular Biology", Springer-Verlag, and NY).

Moreover, after synthesizing a single stranded cDNA by using mRNA obtained by the above-mentioned method as a template and reverse transcriptase, a double stranded cDNA is prepared from this single stranded cDNA. It can be conducted by a method such as the S1 nuclease method (Efstratiadis, A. et al. (1976) Cell 7, 279-288), the Land method (Land, H. et al. (1981) Nucleic Acids Res. 9.2251-2266), the O. Joon Yoo method (Yoo, O. J. et al. (1983) Proc. Natl. Acad. Sci. USA 79, 1049-1053) or the like. However, the Okayama-Berg method (Okayama, H. and Berg, P. (1982) Mol. Cell. Biol. 2, 161-170) is preferable for the purpose of the present invention.

Then, a recombinant phage having a cDNA fragment can be stably maintained and amplified by incorporating the resultant cDNA fragment into a lambda phage vector and allowing it to self-replicate. For example, when lambda phage λZAPII (manufactured by Stratagene corporation) is used, Escherichia coli XL1-Blue MRF' or JM 109 is allowed to make a plaque. The recombinant can be selected based on the existence of coloring due to the metabolism of 5-Bromo-4-chloro-3-indolyl-β-D-galactopyranoside (X-gal) in the plaques. As a vector, not only the lambda phage vector but also a plasmid vector can be used.

Moreover, various commercially available cDNA libraries (for example, manufactured by Clonetech corporation) can also be used. As a method for selecting a clone which has a cDNA encoding the desired protein which has a ceramide kinase activity from the library obtained as mentioned above, any one of the various methods as shown below, for example, can be employed.

### (1) A screening method using a synthetic oligonucleotide probe

If the amino acid sequence of the full length or part of the desired protein has been elucidated (the sequence can be that in any region of the protein, as long as it is specific and containing continuous amino acids), oligonucleotides corresponding thereto may be synthesized (For an amino acid having codon degeneracy, a codon which is highly frequent in the host can be used, or any possible codons corresponded to said amino acid can be used to synthesize the mixture of more than one nucleotides. In the latter case, the number of oligonucleotides to be used can be reduced by incorporating inosine thereinto), and used as a probe after being labeled with ³²P, ³⁵S, biotin or the like, hybridizing with recombinant phage DNA immobilized on a nitrocellulose or a nylon filter to search and select a positive clone.

### (2) A screening method using the probe prepared by polymerase chain reaction

When the amino acid sequence of the full length or part of the desired protein has been elucidated, an oligonucleotide of the antisense strand chain corresponding to a part of the C terminus thereof is synthesized as well as the sense strand chain corresponding to a part of the amino terminus thereof, and PCR is performed, and then a DNA fragment which encodes the desired protein is amplified. The cDNA synthesized by reverse transcriptase reaction of mRNA derived from a cell producing the protein of the present invention or the genomic DNA can be used as a template of the reaction. Thus, the prepared DNA fragment is labeled with ³²P, ³⁵S, or biotin, and the cDNA library or the genomic library was screened by plaque hybridization or colony hybridization using the DNA fragment as a probe, and the desired clone is selected.

### (3) A screening method by letting another animal cell strain produce the protein which has ceramide kinase activity:

An animal host cell is transformed with a plasmid (a plasmid which is self-replicable and contains transcription promoter region, or which is integrated into the chromosome of an animal cell) which is an expression vector wherein the obtained cDNA as mentioned above is inserted, and is allowed to produce the protein encoded by the cDNA. A strain which has a cDNA encoding the protein of the present invention is selected by measuring ceramide kinase activity in the conditioned medium of the cell culture or the extract of the cell, or by detecting the existence of the protein of the present invention using an antibody that specifically binds to the protein of the present invention and a second antibody to said antibody. Conventional cells such as COS and CHO can be used as an animal host cell. However, for detecting the protein of the present invention as an exogenous gene product more easily, it is preferable that the host cell itself does not produce the protein of the present invention under a regular culture condition.

### (4) A method using the system of selective hybridization translation:

The cDNA obtainable from the transformant is blotted on a nitrocellulose filter or nylon filter, and mRNA extracted from tissues or cells having an ability to produce the protein of the present invention are allowed to hybridize thereto, and then mRNA hybridizing to the cDNA is dissociated and collected. The collected mRNA is translated into protein by using a protein translation system (for example, injection into an oocyte of a Xenopus, or a cell free system such as a rabbit reticulocyte lysate and wheat germ system), and the ceramide kinase activity of the protein is examined, or the protein is detected by using an antibody to the protein having ceramide kinase activity, to select the desired strain.

Harvest of DNA encoding the protein of the present invention from the desired transformant which is obtained as mentioned above can be performed by known techniques (Maniatis, T., et al., in "Molecular Cloning A Laboratory Manual", Cold Spring Harbor Laboratory, NY, (1982)). For example, the region corresponding to the cDNA can be cut out from plasmid DNA after separating the fraction corresponding to the plasmid DNA from the cells.

Moreover, the gene which encodes the protein of the present invention can also be obtained preferably by the DNA/RNA amplifying method according to PCR method. Especially when full length cDNA can not be obtained from a library, the full length of the cDNA containing the both ends can be obtained by the above-mentioned RACE. The primer used for said PCR method can be suitably designed on the basis of the sequence information of the gene of the present invention, and can be synthesized according to conventional methods.

DNA thus obtained may be sequenced by, for example, Maxam-Gilbert's chemical modification technique [cf. Maxam, A. M. and Gilbert. W. (1980) in "Methods in Enzymology" 65, 499-576], the dideoxy chain termination method using M13 phage [cf. Messing J. and Vieira J. (1982) Gene, 19, 269-276] or the like.

Moreover, automatic DNA sequence analyzers (for example, the model 373A manufactured by Parkin Elmer Japan Applied Biosystems) or the like, utilizing fluorochromes instead of radio-isotopes, can also be used.

The transformed Escherichia coli strain E.coli pCR3.1-CERK1 SANK 70300 carrying the plasmid wherein the cDNA encoding the most preferable protein of the present invention is inserted was deposited internationally on June 2, 2000 at the National Institute of Bioscience and Human-Technology, Agency of Industrial Science and Technology at 1-1-3, Higashi-cho, Tsukuba-shi, Ibaraki-ken, Japan, and was accorded the deposition number FERM BP-7184. Therefore, the gene which encodes the protein of the present invention is obtainable from said strain.

A fragment containing a gene which encodes the protein of the present invention cloned as described above can be inserted into a vector DNA, and used for the transformation of other prokaryotic host cells or eukaryotic host cells. The gene can be expressed in each host cell by introducing a suitable promoter and sequences regarding the expression of phenotype thereinto.

Suitable prokaryotic host cells include, for example, E. coli (Escherichia coli), Bacillus subtilis, and the like. In order to express the desired gene in such host cells, these host cells can be transformed with a plasmid vector containing a replicon, an origin for replication, derived from a species compatible with the host and regulatory sequences. These vectors preferably have sequences capable of giving selectivity of the phenotype to the transformed cell.

For example, K12 strain can be conventionally used as E. coli. Suitable vectors include pBR322 and the pUC-derived plasmids can be used, without being limited thereto. Other known strains and vectors can also be utilized.

Suitable promoters include the tryptophan promoter (trp), the lactose promoter (lac), the tryptophan lactose promoter (tac), the lipoprotein promoter (lpp), and the polypeptide chain elongation factor Tu promoter (tufB) can be used for producing the protein of the present invention, without being limited thereto.

A preferred strain of Bacillus subtilis is 207-25 strain, and a preferred vector is pTUB228 [cf. Ohmura, K., et al., (1984), J. Biochem., 95, 87-93], without being limited thereto.

If the DNA sequence encoding the signal peptide sequence of Bacillus subtilis α-amylase is linked to the DNA of the present invention, it becomes possible to express the protein of the present invention outside the cells via the secretion thereof.

Examples of the eukaryotic host cells include vertebrate cells, insect cells, and yeasts or the like. Examples of vertebrate cells include: COS cells (Gluzman, Y.(1981) Cell 23, 175-182, ATCC CRL-1650) which are cells derived from an ape, a dihydrofolate reductase-deficient strain of chinese hamster ovary cells (CHO cell, ATCC CCL-61), or the like, but it is not limited thereto (Urlaub, G. and Chasin, L.A. (1980) Proc. Natl. Acad. Sci. USA 77, 4126-4220).

As expression promoters for vertebrate cells, those comprising: a promoter which is usually located in the upstream region of a gene to be expressed; a splicing site of RNA; a polyadenylation site; and a transcriptional termination sequence can be used. It can also comprise an origin of replication if necessary. An example of said expression vector is pSV2dhfr containing the SV40 early promoter (Subramani, S., et. al. (1981), Mol. Cell. Bio., 1, 854-864), without being limited thereto.

When COS cells are used as host cells, expression vectors suitably comprise the SV40 replication origin which enables the cell to self-replicate, a transcription promoter, a transcription termination signal and an RNA splicing site. The expression vectors can be used to transform COS cells by a method, such as the diamino ethyl (DEAE)-dextran method [cf. Luthman. H, and Magnusson. G (1983), Nucleic Acids Res., 11, 1295-1308], the calcium phosphate -DNA co-precipitation method [Graham, F. L. and Van der Eb, A. J., (1973), Virology, 52, 456-457] and the electric pulse electroporation method [cf. Neumann, E., et. al., (1982), EMBO J, 1, 841-845] or the like, and then the desired host cell can be obtained. In the case that CHO cells are used as the host cells, the transformed cells stably producing the protein of the present invention can be obtained by co-transfecting with an expression vector and a vector which can express the neo gene which functions as a marker for antibiotic G418-resistance, for example, PRSVneo (Sambrook, J. et al. (1989): "Molecular Cloning A Laboratory Manual" Cold Spring Harbor Laboratory, NY), pSV2-neo (Southern, P. J. and Berg, P. (1982) J. Mol. Appl. Genet. 1, 327-341) or the like, and selecting G-418 resistant colonies.

In the case that insect cells are used as host cells, established cells (Sf-9 or Sf-21) derived from ovarian-cells of Spodoptera frugiperda which belongs to Lepidoptera Phalaenidae and High Five cells derived from egg cells of Trichopusiani (Wickham, T. J. et al. (1992) Biotechnol. Prog. I: 391-396) or the like are conventionally used as host cells. The pVL1392/1393 utilizing the polyhedrin protein promoter of autographa polyhedron virus (AcNPV) is conventionally used as a baculovirus transfer vector (Kidd, I. M. and V. C. Emery (1993) The use of baculoviruses as expression vectors, Applied Biochemistry and Biotechnology 42,137-159). Besides it, a vector utilizing a promoter of P10 or the basic protein of baculovirus is also available. Furthermore, it is possible to express recombinant protein as a secretory protein by joining the secretion-signal sequence of the envelope surface protein GP67 of AcNPV to the N-terminus of the desired protein (Zhe-mei Wang, et al. (1998) Biol. Chem., 379, 167-174).

As an expression system using a eukaryotic microorganism as a host cell, yeast is generally well-known, and Saccharomyces yeasts, for example, baker's yeast, Saccharomyces cerevisiae, and petroleum yeast, Pichia pastoris are preferable. As an expression vector of eukaryotic microorganisms such as yeasts, the promoter of the alcohol dehydrogenase gene (Bennetzen, J. L. and Hall, B. D. (1982) J. Biol. Chem. 257, 3018-3025), the promoter of the acid-phosphatase gene (Miyanohara, A. et al. (1983) Proc. Natl. Acad. Sci. USA 80, 1-5), or the like can be used preferably. In order to express as a secreting protein, it is possible to be also expressed as a recombinant protein which has a secretion signal sequence and the cleavage site at the N-terminus thereof for an endogenous protease which the host cell has or a known protease. For example, it is known that an active form of human mast cell tryptase, a trypsin-type serin protease, can be secreted into a medium by connecting the secretion signal sequence of the alpha factor derived from yeast and the cleavage site for KEX2 protease to the N-terminal part of the tryptase, and subsequentely expressing in an expression system of petroleum yeast. (Andrew, L. Niles, et al. (1998) Biotechnol. Appl. Biochem. 28,125-131).

Transformants obtained by the above-mentioned methods can be cultured according to conventional methods, and thereby protein of the present invention can be produced either in or outside said transformants. Suitable culture media include various conventionally used media, depending on the host chosen. For example, for COS cells, there can be used RPMI-1640 and Dulbecco's Modified Eagle Medium (hereinafter referred to as DMEM), which can be supplemented with, if necessary, serum component such as fetal bovine serum.

The protein expressed as described above may be isolated and purified by various methods for separation according to the physical and chemical properties thereof (See "the biochemistry data book II", 1175 -1259, the 1st edition, 1st printing, June 23, 1980 published by Tokyokagakudojin; Biochemistry, vol.25, No.25, p8274-8277 (1986); and Eur.J.Biochem., 163, and P313-321 (1987)". For example, specific methods for separation include: common reconstruction treatment; treatment using protein precipitating-agents (salting out); centrifugation; osmotic shock method, freezing and melting method, ultrasonic lysis; various methods of chromatography such as ultrafiltration, gel filtration, adsorption chromatography, ion exchange chromatography, affinity chromatography and high performance liquid chromatography (HPLC), dialysis and combinations thereof.

Especially, since there is a sequence (the amino acid sequence shown in 422-435 of SEQ ID No. 2 of the Sequencing Listing) corresponding to a sequence motif known as the calmodulin binding sequence "the 1-8-14 motif type B (Rhoads, A.R. and Friedberg, F, The FASEB Journal 11, 331-340 reference)" in the amino acid sequence of the protein of the present invention, it is possible to allow a sample containing the protein of the present invention to adsorb on an affinity-chromatography resin which has calmodulin as a substituent (for example, calmodulin-Sepharose), and the resin is then washed with a solvent which does not contain calmodulin, followed by elution with a solvent containing EGTA or EDTA, to purify and collect the protein of the present invention efficiently.

Moreover, it can be purified efficiently in a nickel affinity column by connecting a histidine tag consisting of six residues to the recombinant protein to be expressed.

The protein of the present invention can be easily manufactured in large scale with high yield and high purity by combining the above-mentioned methods.

The amino acid sequence of the protein of the present invention purified as mentioned above can be confirmed by using automatic protein amino acid sequence analyzers (for example, Model 492 manufactured by Perkin Elmer Japan applied biosystems).

In order for the protein obtained from DNA of the present invention by the genetic engineering-procedure to exhibit ceramide kinase activity, it is not necessary for the protein to consist of a sequence which is completely the same as the amino acid sequence shown in the amino acid number 1-537 of SEQ ID No. 2 of the Sequencing Listing. For instance, a partial sequence thereof is also included in the protein of the present invention as long as it exhibits ceramide kinase activity. And, DNA encoding said protein is also included in the present invention.

In general, it is thought that genes of a eukaryotic organism can show polymorphism, as known for the interferon gene or the like. (See, for example, Nishi, T. et al. (1985) J. Biochem. 97, 153-159). As results of the polymorphism, one or more than one of the amino acids can be replaced in some cases. In other cases, a replacement in a nucleotide sequence may result in no change in the corresponding amino acid sequence. Many of the proteins wherein one or more amino acid residues are deleted, added, inserted or replaced in one or more than one positions in the protein of the present invention comprising an amino acid sequence shown in the amino acid numbers 1-537 of SEQ ID No. 2 of the Sequencing Listing, have a ceramide kinase activity (as an example showing that the protein which has the amino acid sequence wherein amino acids are replaced in the natural type amino acid sequence and which has an activity equivalent to natural type protein, it is known that a protein obtained on the basis of an interleukin-2 (IL-2) gene wherein a nucleotide sequence corresponding to a cysteine thereof is replaced with that corresponding to serine keeps its IL-2 activity (Wang, A. et al. (1984) Science 224, 1431-1433)). All of these proteins are included in the present invention, as long as they have a ceramide kinase activity. DNA consisting of equivalent nucleotide sequences encoding these proteins is also included in the present invention.

Those various DNAs of the present invention can also be produced by chemical synthesis based on information of the above-mentioned protein having a ceramide kinase activity, according to a conventional method such as the phosphite triester method (Hunkapiller, M. et al. (1984) Nature 310, 105-111) or the like.

The selection of the codon corresponding to a desired amino acid can be done at random and, for example, it can be determined according to a conventional method, taking the frequency of codons in the host to be used into account (Grantham, R. et al. (1981) Nucleic Acids Res. 9, 143-174). Partial modification of the codon in the nucleotide sequence can be accomplished by site-directed mutagenesis utilizing synthetic oligonucleotide primers encoding the desired modifications [cf. Mark, D. F., et al., (1984), Proc. Natl. Acad. Sci. USA, 81, 5662-5666] or the like, by conventional techniques. Constructing a mutant wherein one or more than one amino acids in an amino acid sequence are deleted may be performed, for example, in accordance with a method of deleting DNA from the terminal using exonuclease Bal31 or the like (Toshimitsu Kishimoto et al., "Zoku-Seikagaku Jikken Kouza 1, Idenshi-Kenkyu-hou II" 335-354), or the cassette mutagenesis method [cf. Toshimitsu Kishimoto, "Shin-Seikagaku Jikken Kouza 2: Kakusan III Kumikae DNA Gijutsu", 242-251].

The protein having ceramide kinase activity as described above is preferably those comprising 537 amino acids of an amino acid sequence shown in SEQ ID No. 2 of the Sequencing Listing wherein the amino terminus thereof is the methionine residue having the amino acid number 1.

Whether DNA can hybridize to DNA comprising the nucleotide sequence shown in the nucleotide numbers 124-1734 of SEQ ID No. 1 of the Sequencing Listing can be determined, for example, by using a probe DNA which is labeled with (α-³²P)dCTP or the like, according to the random primer method [cf. Feinberg, A. P. and Vogelstein, B. (1983), Anal. Biochem., 132, 6-13], or to the nick translation method [cf. Maniatis, T., et al., (1982), in "Molecular Cloning A Laboratory Manual", Cold Spring Harbor Laboratory, NY] or the like. DNA used for hybridization is adsorbed onto nitrocellulose or nylon membrane, and then fixed by heating or UV irradiation. The membrane is next soaked in prehybridisation solution containing 6 x SSC, 5% v/v Denhardt solution and 0.1% v/v sodium dodecyl sulphate (hereinafter referred to as SDS), and incubated at 55°C for 4 hours or more. Then, the probe previously prepared is dissolved in similar prehybridisation solution to a final specific activity of 1 x 10⁶ cpm/ml, followed by incubation at 60°C overnight. Subsequently, the membrane is washed 5 times at 57°C for 5 minutes and further at 57°C for 20 minutes, and is then subjected to autoradiography. By using such a method, cDNA hybridizing to the DNA comprising the nucleotide sequence shown in the nucleotide numbers 124 to 1734 of SEQ ID No. 1 of the Sequencing Listing can be isolated from any cDNA library of various animal cells (Maniatis, T., et al., (1982), "Molecular Cloning A Laboratory Manual", Cold Spring Harbor Laboratory, NY).

As DNA which encodes the protein of the present invention above, DNA which comprises a nucleotide sequence shown in the nucleotide numbers 1 to 4463 of SEQ ID No. 1 of the Sequencing Listing is preferable, and DNA which comprises a nucleotide sequence shown in the nucleotide numbers 124 to 1734 of SEQ ID No. 1 of the Sequencing Listing is more preferable.

Whether the protein of the present invention has ceramide kinase activity can be confirmed according to the following method wherein the Spiegel method (See Olivera, A. and Spiegel, (1993) Nature 365, 557 to 560) for measuring sphingosine kinase activity using radioactive ³²P-ATP is modified.

Specifically, the solution containing the protein, HEPES buffer solution (pH 7.0) containing magnesium chloride, EDTA, dithiothreitol, sodium vanadate, and protease inhibitor (for example, Complete (trademark), manufactured by Boehringer Manheim), C₆ ceramide dissolved in a substrate [bovine serum albumin (hereinafter referred to as "BSA") solution having a concentration of 4 mg/ml or ceramide micellized by using octyl glucoside (a preparation purified from bovine fetal brain, manufactured by Sigma), and radio-labeled ³²P-ATP are mixed and incubated at 37°C to perform a ceramide kinase reaction. Then, the reaction is stopped by adding IN hydrochloric acid thereto and chloroform: methanol: concentrated hydrochloric acid (100:200:1 v/v) is added thereto and extracted. The resultant lower layer (chloroform phase) is developed on thin layer chromatography (hereinafter referred to as "TLC"), and analyzed. The ceramide kinase activity can be determined by quantifying the spot in said TLC corresponded to ceramide-1-phosphate (hereinafter referred to as "Cer-1-P") which is a product.

However, the method for detecting a ceramide kinase activity which the protein of the present invention has is not restricted thereto, but it is also possible to use other substrates which can be phosphorylated limitedly by the protein of the present invention (for example, ³H-, ¹⁴C- radio-labeled ceramide, or various fluorescent-labeled ceramides, or the like).

Moreover, the evaluation or screening of ceramide kinase activators or inhibitors can be conducted, by allowing a sample to be tested such as a synthesized compound or an extract from a microorganism culture or the like to be brought together, and determining whether the sample to be tested activates or inihibits the ceramide kinase activity of the protein of the present invention, in the above-mentioned ceramide kinase activity detection method. For example, the amount of a substrate specifically phosphorylated by the protein in the presence of the compound during substrate phosphorylation reaction (referred to as "a") is compared with the amount of the substrate specifically phosphorylated by the protein in the absence of the compound (referred to as "b"). If a is larger than b, the compound can be determined to posses an activity of activating ceramide kinase. On the contrary, if a is smaller than b, the compound can be determined to posses an activity of inhibiting ceramide kinase. Especially, a substance which strongly and specifically activates the ceramide kinase activity of the protein of the present invention, but does not influence the activity of other phosphorylation enzymes (kinases) can be expected to have a pharmaceutical effect as a ceramide kinase specific activator in the various above-mentioned diseases (various inflammatory disorders, HIV infection, type 2 diabetes mellitus, obesity, septicemia, arteriosclerosis, or the like). On the other hand, a substance which strongly and specifically inhibits the ceramide kinase activity of the protein of the present invention, but does not influence the activity of other phosphorylation enzymes (kinases) can be expected to have a pharmaceutical effect as a ceramide kinase specific inhibitor for the above-mentioned uses (improvement in the treatment effect for the radiotherapy and chemotherapy of cancer or the like).

An example of an antibody which specifically binds to the protein of the present invention can be a monoclonal antibody which specifically binds to the protein of the present invention, which can be obtained by the following method.

Preparation of a monoclonal antibody generally requires the following steps:
(a) purification of a biomacromolecule for use as the antigen;
(b) preparation of antibody producing cells, after immunizing an animal using injections of the antigen, bleeding the animal and assaying the antibody titer, in order to determine when the spleen is removed;
(c) preparation of myeloma cells;
(d) fusing the antibody producing cells with myeloma cells;
(e) selecting a hybridoma producing the desired antibody;
(f) preparing a single cell clone (cloning);
(g) optionally, culturing the hybridoma cells, or growing animals wherein the hybridoma cells have been transplanted, for large scale preparation of the monoclonal antibody; and
(h) testing the biological activities and the recoginition specificity, or assaying properties as a marker reagent, of the monoclonal antibody thus prepared.

The method for the preparation of a monoclonal antibody is described below in more detail, in line with the above described steps. However, the method for preparing the antibody is not limited thereto. Antibody producing cells other than spleen cells and myeloma cells can also be used.

### (a) Purification of antigen

The proteins of the present invention prepared as described above or a part thereof can be used as an antigen. Furthermore, since the primary structure of the protein of the present invention is disclosed in the present invention, a partial peptide thereof can be chemically synthesized by a method well-known to the person skilled in the art, and used as the antigen.

### (b) Preparation of antibody producing cells

The antigen produced in step (a) is mixed with an adjuvant such as Freund's complete and incomplete adjuvant, or supplementing agent such as potassium alum, and an experimental animal is administrated with said antigen as an immunogen. The most preferable experimental animal for use is a mouse, without being limited thereto.

Suitable administration routes for immunizing the mouse include the subcutaneous, intraperitoneal, intravenous, intradermal and intramuscular injection routes, with subcutaneous and intraperitoneal injections being preferred.

Immunization can be done once, or several times, repeatedly with appropriate intervals (preferably 1 to 5 weeks per interval). Immunized animals are monitored for antibody titer in their sera, and an animal exhibiting a sufficiently high antibody titer is selected as the source of antibody producing cells. Selection of an animal exhibiting a high titer allows the subsequent process to be more efficient. Cells for the subsequent fusion are generally harvested from the animal 3 to 5 days after the final immunization.

Methods for assaying antibody titer include various well known techniques such as radioimmunoassay (hereinafter, referred to as RIA), enzyme-linked immunosorbent assay (hereinafter, referred to as ELISA), fluorescent antibody assay and passive hemagglutination assay, with RIA and ELISA more preferred for the detection sensitivity, the rapidity, the accuracy, and the potential for automation.

Determination of antibody titer can be performed, for example, by ELISA, as follows. First, purified or partially purified antigen is adsorbed onto the surface of a solid phase, such as a 96-well ELISA plate, followed by blocking any remaining surface wherein antigen is not bound, with a protein unrelated to the antigen such as bovine serum albumin (hereinafter referred to as BSA). After washing, the well surfaces are contacted with a sample diluted stepwise as the first antibody (for example, mouse serum) to enable binding of the monoclonal antibody in the samples to the antigen. An enzyme-labeled anti-mouse antibody as the secondary antibody, is added to be bound to the mouse antibody. After washing, the substrate for the enzyme is added, and antibody titer can then be calculated by measuring the absorbance change due to color development which is caused by the decomposition of the substrate or the like.

### (c) Preparation of myeloma cells

In general, cells from established mouse cell lines serve as the source of myeloma cells, for example, 8-azaguanine resistant mouse (BALB/c)-derived myeloma strains P3X63Ag8U.1 (P3-U1) [Yelton, D. E., et al., Current Topics in Microbiology and Immunology, 81, 1-7, (1978)], P3/NSI/1-Ag4-1(NS-1) [Kohler, G, et al., European J. Immunology, 6, 511-519 (1976)], Sp2/0-Ag14 (SP-2) [Shulman, M., et al., Nature, 276. 269-270 (1978)], P3X63Ag8.653 (653) [Keamey, J. F., et al., J. Immunology, 123, 1548-1550 (1979)] and P3X63Ag8 (X63) [Horibata, K. and Hams, A. W., Nature, 256, 495-497 (1975)]. The cell lines are subcultured in an appropriate medium such as 8-azaguanine containing medium [RPMI-1640 medium supplemented with glutamine, 2-mercaptoethanol, gentamicin, fetal calf serum (hereinafter referred to as FCS), and 8-azaguanine], Iscove's Modified Dulbecco's Medium (hereinafter referred to as IMDM) or Dulbecco's Modified Eagle Medium (hereinafter referred to as DMEM). The cells are then transferred to a normal medium such as ASF104 medium (Ajinomoto, K. K.) containing 10% FCS, 3 to 4 days prior to cell fusion, and maintained so that at least 2 x 10⁷ cells are available just on the day of cell fusion.

### (d) Cell fusion

The antibody producing cells to be used are plasma cells and lymphocytes which are the precursor cells thereof, which can be obtained from any part of an individual. Typically, they can be obtained from spleen, lymph nodes, peripheral blood, or any appropriate combination thereof, and spleen cells are most generally used.

After the last immunization, tissue wherein antibody producing cells are present, such as the spleen, is removed from mouse exhibiting the predetermined antibody titer, and spleen cells as antibody producing cells are prepared. The currently most favorable technique for fusing the spleen cells with the myeloma cells obtained in step (c), is a method using polyethylene glycol which shows relatively low cytotoxicity, and the procedures thereof are simple. An example of this technique is as follows.

The spleen cells and myeloma cells are washed well with serum-free medium (such as RPMI 1640) or phosphate buffer saline (hereinafter referred to as PBS), and then mixed, so that the ratio of the number of spleen cells to that of myeloma cells is approximately between 5 : 1 and 10:1, and then centrifuged. After the supernatant is discarded and the pelleted cells are sufficiently dispersed, 1 ml of serum-free medium containing 50% (w/v) polyethylene glycol (m.w. 1,000 to 4,000) is added thereto dropwise with mixing. Subsequently, 10 ml of serum-free medium is slowly added and then centrifuged. The supernatant is discarded again, and the pelleted cells are suspended in an appropriate amount of a normal medium containing hypoxanthine - aminopterin - thymidine (hereinafter referred to as "HAT") solution and mouse interleukin-2 (hereinafter referred to as IL-2). The suspension is then dispensed into the wells of culture plates (also referred herein simply as "plates") and incubated in the presence of 5% CO₂ at 37 °C for about 2 weeks, with the supplementary addition of HAT medium as appropriate.

### (e) Selection of hybridomas

When the myeloma cell as mentioned above is a strain resistant to 8-azaguanine, i.e., it is deficient in hypoxanthine guanine phosphoribosyl transferase (HGPRT), any unfused myeloma cells and any myeloma-myeloma fusions are unable to survive in the HAT medium. On the other hand, fusions of antibody producing cells with each other, as well as hybridomas of antibody producing cells with myeloma cells can survive, but the former only having a limited lifetime. Accordingly, continuous culture in HAT medium results in the survival of only the hybridomas wherein an antibody producing cell and a myeloma cell are fused, and the hybridoma can be selected.

The resulting hybridomas grown up into colonies are then transferred into HAT medium lacking aminopterin (hereinafter referred to as HT medium). Thereafter, for example, an aliquot of the conditioned medium is collected to determine antibody titer using ELISA.

### (f) Cloning

The hybridoma which is revealed to produce a specific antibody by measuring the antibody titer according to a method similar to that described in the step (b), is then transferred to another plate for cloning. Suitable cloning methods include: the limiting dilution method wherein hybridomas are diluted to be one cell per well of a plate and then cultured; the soft agar method wherein colonies are recovered after culturing in soft agar medium; the method of using a micromanipulator to separate a single cell for culture; and "sort-a-clone" wherein a single cell is separated by a cell sorter. The limiting dilution method is simple and is commonly used.

The cloning procedure according to, for example, the limiting dilution method is repeated 2 to 4 times on each well wherein the antibody titer is detected, and the clones exhibiting stable antibody titers are selected as the monoclonal antibody producing hybridomas of the present invention.

### (g) Preparation of monoclonal antibody through the culture of the hybridoma:

The hybridoma obtained by the cloning is then cultured in normal medium which is replaced with HT medium. Large-scale culture can be performed by rotating culture using large culture bottles, or by spinner culture. The supernatant from the large-scale culture is applied to be purified by a suitable method, such as gel filtration, which is well known to those skilled in the art, and a monoclonal antibody specifically binding to the protein of the present invention can be obtained. The hybridoma may also be grown intraperitoneally in a mouse having the same phyletic lineage such as BALB/c mouse, or in Nu/Nu mouse, to obtain ascites containing a large amount of the monoclonal antibody of the present invention. Easier purification can be conducted through the use of commercially available monoclonal antibody purification kits (for example, MAbTrap GII Kit; Pharmacia).

The monoclonal antibody prepared as mentioned above has high antigen specificity to the protein of the present invention.

### (h) Assay for monoclonal antibody

Determination of the isotype and the subclass of the monoclonal antibody obtained can be performed as follows. Suitable identification methods include the Ouchterlony method, ELISA and RIA. The Ouchterlony method is simple, but it is necessary to concentrate the solution when the concentration thereof is low. When ELISA or RIA is used, the conditioned medium can be reacted directly with an antigen adsorbed on a solid phase and further can be reacted with secondary antibodies having specificity for the various immunoglobulin isotypes and subclasses, and then the isotype and subclass of the monoclonal antibody can be identified. Commercially available kits for identification such as a Mouse Typer Kit (manufactured by BioRad) can be used with simpler methods.

Furthermore, the quantification of protein can be performed according to Folin-Lowry's method, or by calculation based on the absorbance at 280 nm [1.4 (OD 280) = Immunoglobulin 1 mg/ml].

The monoclonal antibody thus obtained can be used for detecting, separating and purifying the protein of the present invention based on its specificity.

The polynucleotide comprising the nucleotide sequence which encodes the protein of the present invention can be used for gene therapy of diseases (various inflammatory disorders, HIV infection, type 2 diabetes mellitus, obesity, septicemia, arteriosclerosis, or the like) which are effectively treated or prevented by ceramide kinase activity of the protein. In said gene therapy, for example, the polynucleotide comprising the nucleotide sequence which encodes the protein of the present invention is inserted into a virus vector, and a patient is infected with the virus (which has been detoxicated) having the recombinant virus vector. Since the protein of the present invention is produced and ceramide kinase activity is exhibited in the patient body, the symptoms can be improved, or the progress thereof can be attenuated in the above-mentioned disorder.

As a method for introducing the gene therapeutic agent into cells, either a method for introducing the gene using a virus vector or a non-viral method for introducing the gene can be adopted (Nikkei Science, April, 1994, 20-45; Jikkenigaku Zoukan, 12(15)(1994); Jikkenigaku Bessatu, "Idenshichiryo no Kisogijutu", Yodosha, (1996)).

Examples of a method for introducing a gene using a virus vector can be the method comprising inserting DNA encoding TR4 or variant TR4 into a DNA virus or RNA virus such as a retrovirus, adenovirus, adenovirus related virus, Herpesvirus, vaccinia virus, poxvirus, poliomyelitis virus, and sindbis virus or the like. Among these, a method using retrovirus, adenovirus, adenovirus related virus and vaccinia virus is especially preferable. Examples of non-viral methods include the method comprising administering an expression plasmid directly into muscle (DNA vaccine method), the liposome method, the lipofectin method, the microinjection method, the calcium phosphate method, the electroporation method, or the like, and the DNA vaccine method and the liposome method are preferable.

A gene therapeutic agent can be effective as a medicament if it is introduced by an in vivo method comprising introducing DNA directly into the body or by an ex vivo method wherein some kinds of cells are taken out from a human, and DNA is introduced thereinto outside the body, and then the cells are returned to the body (Nikkei Science, April 1994, 20-45; Gekkanyakuji, 36(1), 23-48 (1994); Jikkenigaku Zoukan, 12 (15), (1994)).

For example, when a gene therapeutic agent is administered to a patient by the in vivo method, it is done through a suitable route such as intravenous, intraarterial, hypodermical, intradermical and intramuscular or the like, depending on the disease or the symptom or the like. Moreover, while said gene therapeutic agent is generally formulated as an injection or the like when it is administrated by the in vivo method, conventional carriers can be added thereto if necessary. Moreover, when it is formulated in the form of a liposome or membrane-fusion liposome (Sendai-virus liposome or the like), it can be formulated as a liposome preparation such as a suspended agent, cryogen, and cryogen for centrifugation and subsequent concentration or the like.

Anucleotide sequence complementary to a partial sequence of the nucleotide sequence shown in SEQ ID No. 1 of the Sequencing Listing can be used for so-called anti sense treatment. An antisense molecule may be used as DNA which usually consists of a 15 or 30 mer complementary to a part of the nucleotide sequence shown in SEQ ID No. 1 of the Sequencing Listing, or a stable DNA derivative such as a phosphorothioate, methyl phosphonate, or a morpholino derivative or the like, or a stable RNA derivative such as 2'-O-alkyl RNA. Such an antisense molecule can be introduced into a cell by a method well known in the technical field of the present invention, such as microinjection, liposome capsulation, or expression using a vector having an antisense sequence or the like. In radiotherapy and chemotherapy of cancer, for example, the therapeutic efficacy can be expected to be improved when the activity of the protein encoded by the nucleotide sequence shown in the nucleotide numbers 124 to 1734 of SEQ ID No. 1 of the Sequence Listing is reduced by the antisense therapy.

A composition useful as a medicament containing the above-mentioned antisense oligonucleotide may be manufactured according to well-known methods such as mixing with a pharmaceutically acceptable carrier. Examples of such carriers and manufacture methods are described in Pharmaceutical Sciences by Remington. A sufficient amount of the medicament for treating hyperlipidemia, wherein the expression of the gene containing the nucleotide sequence shown in the nucleotide numbers 124 to 1734 of SEQ ID No. 1 or the activity of the gene product is found to be abnormal, is administered to each individual. The effective dose may be varied due to various factors such as conditions, weight, sex, and age, and due to difference in the administration method such as hypodermical, local, oral and intramuscular. For example, it can be administrated at 0.02 to 0.2 mg/kg/hour for 2 hours when it is done by the intravenous injection, and at 1 to 200 mg/m²/day in the case of hypodermical administration.

### [Best Mode of Carrying Out the Invention]

The present invention is explained below in more detail with reference to examples, but the present invention is not limited thereto. In addition, in the following examples, each operation by genetic manipulation was conducted according to a method described in 1989 "Molecular Cloning" [Sambrook, J., Fritsch, E.F. and Maniatis, T. work, and Cold Spring Harbor Laboratory Press], unless otherwise especially indicated. Alternatively, if a commercially available reagent and a commercially available kit were used according to the directions thereof.

### [Example 1] cDNA cloning

### 1) Search for cDNA clone having low homology to mouse sphingosine kinase 1 and analysis of a nucleotide sequence

Homology search was performed by using the algorithm of tblastn to the dbEST database of NCBI based on the known amino acid sequence of mouse sphingosine kinase 1, and an EST clone (GenBank accesion number AA355581) which shares the homology with mouse sphingosine kinase 1 was found.

In order to obtain a cDNA clone which encodes the nucleotide sequence of this EST clone, library screening was performed on the basis of the method described below using a probe produced by PCR.

### 2) Amplification of the cDNA fragment by PCR

The following two oligonucleotide primers based on the EST clone AA 355581 were synthesized: 
5 '-tcaccactgacatcatcgttactgaacatgcta-3'(cerk1 : SEQ ID No. 3 of the Sequencing
Listing);
 and

5'-caacgatatgcagcgccgaggtttctgcgtcgctg-3' (cerka1 : SEQ ID No. 4 of the Sequencing
Listing).

Subsequently, PCR was performed with the LA PCR kit version 2.1 (Takara Shuzo Co., Ltd., Japan) by using a commercially available cDNA library as a template. Namely, 50 µl of reaction mixture consisting of 5 µl of cDNA library (Marathon-Ready human leukemia (manufactured by Clontech corporation)), 1 x LA PCR buffer solution containing 0.4 µM each of oligonucleotide primer (cerk1 and cerka1), 400 µM each of dATP, dGTP, dCTP and dTTP, 2.5 mM magnesium chloride and 0.05 units of LA Taq DNA polymerase (included in the kit) were prepared. First, the reaction mixture was heated at 94°C for 2 minutes, after that a cycle consisting of heating at 94°C for 1 minute, at 60°C for 1 minute and at 72°C for 2 minutes, was repeated 30 times. And then, the reaction solution was heated at 72°C for 10 minutes. Those heating steps were performed by using Takara PCR thermal cycler MP (manufactured by Takara Shuzo Co., Ltd., Japan). As a result of analyzing this reaction mixture by 1% agarose gel electrophoresis, a band having a size near 300bp which the desired DNA fragment would have was observed. The piece of the gel containing this band was cut out, and the DNA fragment in the band was collected from the piece by using a kit for extraction (QIA Quick Gel Extraction Kit manufactured by Qiagen). The obtained DNA fragment was inserted in a pCR2.1 vector (inserted with a TA cloning Kit for Eukaryotes manufactured by Invitrogen Corporation) according to the T/A cloning process (Clark and J. M. et al.(1988) Nucleic Acid Res.16: 9677-9686), and was introduced into the Escherichia coli TOP10F' strain (attached to TA cloning Kit for Eukaryotes). The plasmid DNA pCR2.1-CERKM was extracted from the obtained transformant, and the nucleotide sequence was determined for all the nucleotide sequences of cDNA inserted according to the dideoxy nucleotide chain termination method. Consequently, the obtained nucleotide sequence was that encoding a part of the nucleotide sequence of the above-mentioned EST clone AA 355581 (nucleotide numbers 611-910 of SEQ ID No. 1 of the Sequencing Listing).

### 3) Preparation of a filter for plaque hybridization

Library screening was performed by using a commercially available cDNA library (Lambda ZAP II library manufactured by Stratagene corpozation).

First, the titer of a Lambda ZAP II library was determined by the method described below. The Escherichia coli XL-1 Blue MRF' strain was cultured at 37 °C in LB culture medium (a culture medium wherein 10 g of sodium chloride, 10 g of bactotrypton, and 5 g of yeast extract were dissolved in 1 l of water and the pH was adjusted to 7.0) containing 10 mM of magnesium sulfate and 0.2 % of maltose. Escherichia coli cells were collected by centrifugation at the logarithmic phase of the growth, and 10 mM magnesium sulfate solution added thereto, and then suspended so that the absorbance (OD 600) at 600 nm was 0.5. The phage containing solution was diluted to 10⁴ to 10⁷ times with TE buffer solution, and 1 µl of each diluent was mixed with 200 µl of the Escherichia coli suspension, and incubated at 37 °C for 15 minutes, and the soft-agar culture medium [LB culture medium (a culture medium wherein 10 g of sodium chloride, 10 g of bactotrypton, and 5 g of yeast extract were dissolved in 1 l of water and the pH was adjusted to 7.0) wherein 0.7 % of agar was added.] preheated at 48°C was added, and then inoculated on a sterilized plastics laboratory dish. After incubation at 37°C for 6 to 8 hours, the titer was calculated by counting the plaques. The resulting titer of the primary library was 4.6 x 10⁶ plaque forming units (hereinafter referred to as "pfu").

Since primary libraries are generally unstable, said primary library consisting of 1 x 10⁶ pfu was again used to infect Escherichia coli, and amplified by the same method as described above, and then was collected, following this, the Lambda ZAP II library was obtained.

DNA from 5 x 10⁵ plaques among the Lambda ZAP II libraries was fixed on a nitrocellulose filter (manufactured by Stratagene corporation). That is, the Escherichia coli infected with the phage having a cDNA library was spread so that 5 x 10⁴ plaques per plate with a diameter of 15 cm might be formed. The nitrocellulose filter was put on the plate, and was penetrated at three points on a plate with a needle of 18G for identifying the positions thereof later. The plaques were transferred by leaving it for 2 minutes at room temperature, and then the filter was removed, and soaked in an alkali solution (0.5 M sodium hydroxide, 1.5 N sodium chloride) for 2 minutes, and then soaked in a neutralization solution (1.5 M sodium chloride, 0.5 M Tris-hydrochloric acid (pH 8.0)) for 5 minutes, and further soaked in a washing solution containing 2 x SSC and 0.2 M Tris-hydrochloric acid (pH 7.5) for 30 seconds, and subsequently air-dried completely at room temperature for 2 hours. After air drying, the filter was baked at 80°C for 2 hours in order to immobilize the plaques.

### 4) Preparation of probe and hybridization

The plasmid pCR2.1-CERKM obtained by the above 2) was digested with a restriction enzyme, EcoRI. The resultant inserted DNA fragment (300 bp) was labeled with ³²P by using a DNA labeling kit (Random Primer Labeling System, manufactured by Gibco BRL: Feinberg, A. P. and Vogelstein, B. (1983) Anal. Biochem. 132, 6). Then, the unincorporated nucleotides were removed by using a micro spin column (Probe Quant G-50 Micro Column, manufactured by Pharmacia) which was filled with a gel filtration resin.

On the other hand, the nitrocellulose filter wherein the plaques were transferred as prepared in the above 3) was incubated at 37 °C for 2 hour in a solution containing 50 % formamide, 5 x SSC, 5 x Denhardt solution, 0.1 % SDS and 100 µg/ml denatured salmon sperm DNA (prehybridization). After then, the probe labeled with ³²P was added to be 1 x 10⁶ cpm/filter, and incubated at 37 °C for 12 to 18 hours (hybridization). Then, the filter was washed in a solution containing 1 X SSC, 0.1% SDS at 37 °C for 20 minutes, and then washed in a solution containing 0.1 X SSC, 0.1 % SDS at 60°C for one hour. Then, the filter was subjected to autoradiography.

Consequently, 12 positive plaques were identified at primary screening. After picking up those plaques from the plate and repeating the above-mentioned screening operation for each plaque, seven positive clones were obtained. Escherichia coli SOLR strain was coinfected with each positive clone and with the helper phage (ExAssist which was attached to the Lambda ZAP II Library manufactured by Stratagene corporation), and pBluescript phagemid vector containing the desired cDNA was cut out. The transformed Escherichia coli strain carrying the plasmid wherein the desired cDNA was inserted in the pBluescript phage vector was obtained by the above-mentioned operation. These phage DNA were digested with a restriction enzyme, EcoRI, and subjected to polyacrylamide gel electrophoresis, and the length of the desired cDNA was determined. As a result, three plasmids (pBK-29, pBK-5, and pBK-33) of which the length of inserted cDNAs were about 1.5 kbps, about 3.5 kbps, and about 4.4 kbps respectively were obtained.

### 5) Determination of nucleotide sequence

All the nucleotide sequences of cDNA inserted in plasmids pBK-29, pBK-5, and pBK-33 obtained by the above 4), were determined by the dideoxy nucleotide chain termination method. In addition, parts of the sequences were analyzed by using full automatic DNA sequence analysis apparatus (model 373A, manufactured by Perkin Elmer Japan Applied Biosystems corporation). Consequently, cDNA inserted in pBK-33 had the nucleotide sequence which consists of 4463 bp(s) (SEQ ID No. 1 of the Sequencing Listing) including all of the sequences of the inserted cDNAs of pBK-29 and pBK-5. Furthermore, this nucleotide sequence and the amino acid sequence encoded by the open reading frame (hereinafter referred to as "ORF") included in this nucleotide sequence (amino acid numbers 1-537 of SEQ ID No. 2 of the Sequencing Listing) were subjected to homology search with DNA databases, GenBank and EMBL, and a protein database, SWISS-PLOT. As a result of the homology search, there were no proteins having known function which were especially highly homologous to the amino acid sequence shown in the amino acid numbers 1-537 of SEQ ID No. 2 of the Sequencing Listing, and thus it became clear that it is a novel protein. Moreover, the sequence homology between the amino acid sequence which is shown in the amino acid numbers 1-537 of SEQ ID No. 2 of the Sequencing Listing and the amino acid sequence covering the full length of the human sphingosine kinase 1 or 2 was only 29%, but the conserved domain thereof was highly homologous to those of sphingosine kinases beyond species (See T. Kohama et al. (1998) J. Biol. Chem. 273. 23722-23728). Furthermore, when a motif search was performed, it was suggested that it possessed a PH domain (See A. D. Ma & C. S. Abrams (1999) Thromb. Haemost, 82,399 to 406) and a diacylglycerol kinase domain (See F. Sakana & H. Kanoh (1997) Int. J. Biochem. Cell Biol. 29, 1139-1143) . The cDNA which encoded the novel protein thus obtained was named hCERK1.

### [Example 2] Expression of the recombinant protein

### 1) Production of an expression vector

The hCERK1 obtained in Example 1 was expressed using a vertebrate cell expression system, and expressed protein was obtained. First, a eukaryote expression vector for expressing cDNA which has the cDNA encoding the amino acid numbers 1-537 of the amino acid sequence shown in SEQ ID No. 2 of the Sequencing Listing at the down stream of a cytomegalovirus promoter was constructed.

In order to construct the above-mentioned vector, pBK-33 obtained in 4) of Example 1 was digested with a restriction enzyme, SacII, and a fragment of about 2.3 kbp(s) including the inserted cDNA was isolated. On the other hand, the vector pCR3.1 (manufactured by Invitrogen) for expression in eukaryotes was digested with a restriction enzyme, EcoRI, and dephosphorylated with alkaline phosphatase. The above-mentioned 2.3 kbp fragment and pCR3.1 vector were blunt-ended by using a DNA Blunting Kit (Manufactured by Takara Shuzo Co., Ltd., Japan), and connected to each other by a reaction using T4 DNA ligase (Manufactured by Takara Shuzo Co., Ltd., Japan), and then introduced into the Escherichia coli TOP10F' strain. The plasmid DNApCR3.1-CERK1 was extracted from the obtained transformant, and the full nucleotide sequence of the inserted cDNA was confirmed by the dideoxy chain termination method.

The transformed Escherichia coli strain E.coli pCR3.1-CERK1 SANK 70300 carrying this plasmid pCR3.1-CERK1 for expression was internationally deposited on June 2, 2000 at the National Institute of Bioscience and Human-Technology, Agency of Industrial Science and Technology at 1-1-3, Higashi-cho, Tsukuba-shi, Ibaraki-ken, Japan, and the deposition number FERM BP-7184 was given.

### 2) Expression in animal cells

In order to examine whether the protein of the present invention has ceramide kinase activity, the human embryo kidney cell HEK293 (ATCC CRL-1573) was transfected with the expression vector pCR3.1-CERK1 obtained above. That is, after putting 6 x 10⁵ HEK293 cells per well into a polylysine-coated 6-well plate for tissue culture (manufactured by Iwaki Glass) and incubating at 37 °C for 24 hours, they were transfected with one µg of pCR3.1-CERK1 by using a transfection reagent (Lipofectamin plus, manufactured by Life technology) according to the directions attached thereto. After the operation for the transfection was finished, cells were cultured at 37 °C for one day and the conditioned medium was removed. Then, the cells were collected by using buffer solution A (10 % glycerol, 1 mM dithiothreitol, 1 mM EDTA, 1 mM sodium vanadate, protease inhibitor (Complete™, manufactured by Boeheringer Mannheim), a 200 mM Hepes buffer solution, pH 7.0). By ultrasonication with a probe-type sonicater (Cell Disrupter 200, manufactured by Branson), the collected cells were broken into pieces, and the resulting cell lysate was diluted and then used for measurement of enzyme activity as shown in Example 3.

Since the pCR3.1 expression vector has the neo gene which functions as a marker for antibiotics G418 resistance, G418 resistant colonies were selected by culturing HEK293 cells after transfection of pCR3.1-CERK1 in the presence of G418. Thus, the transformed cell which produces the protein of the present invention stably was obtained.

### [Example 3] Measurement of ceramide kinase activity

Measurement of ceramide kinase activity was performed according to the method described below using the cell lysate containing the protein of the present invention which was prepared in Example 2 above, by using [³²P]-γ-ATP and various ceramides as a substrate.

Specifically, 10 µl of 1 mM C₆-ceramide prepared by using BSA solution (4 mg/ml) and 10µl of a mixture of 100 mM magnesium chloride and 2 mM of radiolabeled [³²P]-gamma-ATP (manufactured by NEN) were added to 180 µl of buffer B [1 mM dithiothreitol, 1 mM EDTA, 1 mM sodium vanadate, protease inhibitor (Complete ™, manufactured by Boeheringer Mannheim), 1 mM calcium chloride, a 200 mM HEPES buffer solution, pH 7.0] which further contains the protein of the present invention prepared in Example 2, and then incubated at 37 °C for 20 minutes to conduct the ceramide kinase reaction. Next, after stopping the reaction by adding 20 µl of 1 N hydrochloric acid, 800 µl (100:200:1, v/v) of chloroform : methanol : concentrated hydrochloric acid, 250 µl of 2.5 M potassium chloride, and 250 µl of chloroform were added thereto and extracted. The resulting extract was subjected to centrifugation at 2000 x g, and the obtained lower layer (chloroform phase) was developed and analyzed on TLC. In addition, as the developing solvent used for TLC, butanol ethanol water acetic acid (80:20:10:20) was found to be preferable. The ceramide kinase activity was determined by quantifying the radioactivity of the spot corresponding to ceramide-1-phosphate (hereinafter referred to as "Cer-1-P") with an imaging analyzer (BAS2000, Fuji film) as a product (identified as the spot which appears stronger with the extract of the reaction using the sample derived from the cell which was transformed with pCR3.1-CERK1 than that which appeared with the extract of the negative-control reaction mixture using a sample derived from the cell which was transformed with an empty vector pCR3.1) in the above TLC.

As a result of measuring by the above-mentioned method, the ceramide kinase activity of the enzyme solution which contains the protein of the present invention obtained in Example 2 was 1280 pmol/min/mg when the substrate was C₆-ceramide. The Km value over C₆-ceramide was 7 µM. In addition, the ceramide kinase activity measured similarly for the cell lysate obtained when HEK293 cell was transfected with only pCR3.1 vector as negative-reaction control was 7 pmol/min/mg.

And, it was more preferable to micellize the ceramide using surface active agents such as an octyl glucoside, when natural type ceramides such as ceramide derived from bovine fetal brain is used as the substrate. That is, a necessary amount of ceramide dissolved in chloroform was dried to be solid under a nitrogen air current, and the buffer solution B which contains 0.2% of octyl glucoside was added thereto, and then subjected to ultrasonication, and finally 100 µM ceramide solution was provided. 90 µl of the mixture of buffer solution B containing the enzyme solution and 10 µl of the mixture consisting of 100 mM magnesium chloride and 2 mM radio-labeled [³²P]-γ-ATP were added to 100 µl of said substrate solution, and then incubated at 37 °C for 20 minutes to perform the ceramide kinase reaction. Extraction of Cer-1-P was conducted by a similar procedure to the above-mentioned. The ceramide kinase activity of the enzyme solution containing the protein of the present invention measured by this method was 200 pmol/min/mg when the substrate was ceramide derived from bovine fetal brain.

Then, C₆-ceramide was used as a substrate and the optimum assay conditions for the protein of the present invention were examined. As a result, the optimum pH of the protein of the present invention was 7 to 7.5, and was activated by magnesium chloride and calcium chloride. Moreover, this ceramide kinase activity was inhibited by surface active agents such as Triton X-100 and cholic acid, phosphatidylserine, and Tris buffer. Moreover, when the cell lysate wherein the protein of the present invention was expressed was divided into a membrane fraction and a cytoplasmic soluble fraction by centrifugation at 100000 x g after the removal of non-lysed cells through centrifugation at 3000 x g, and then the ceramide kinase activity of each fraction was measured, the stronger ceramide kinase activity was observed in the membrane fraction.

Until now, ceramide kinase activity has been reported in nerve synaptic vesicles (See S. M. Bajjalieh, et al. (1989) J. Biol. Chem. 264, 14354 -14360), HL-60 cell (See K. A. Dressier, et al.(1990) J. Biol. Chem. 265, 14917-1492), neutrophil leucocytes (See V. T. Hinkovska-Galcheva, et al. (1998) J. Biol. Chem. 273, 33203-33209 reference) etc. As the properties that they shared, it have been pointed out that they are activated by calcium, that they are membrane-binding type, and that the optimum pH is neutral. The ceramide kinase activity of the recombinant protein of the present invention had all of these properties.

While the protein of the present invention has a diacylglycerol (hereinafter referred to as "DG") kinase domain, it was activated by calcium which does not influence DG kinase (See F.Sakana & H.Kanoh (1997) Int. J. Biochem. Cell Biol. 29, 1139-1143), and was inhibited by phosphatidylserine which is an activator of DG kinase (See H. Kanoh et al. (1883) J. Biol. Chem. 258, 1767-1774). Namely, it had clearly different properties from those DG kinase has.

Next, in order to examine the substrate specificity of the protein of the present invention, the phosphorylation activity of HEK293 cells expressing pCR3.1-CERK1 was compared with that of the cells expressing only pCR3.1 vector (the enzyme activity which HEK293 cell has endogenously), by using various lipids as substrates (Table 1).

**[Table 1]**

| Substrate | Phosphorylation activity (pmol/min/mg) | | Ratio of activities |
|---|---|---|---|
| | Transformed with pCR3.1-CERK1 | Transformed with pCR3.1 | (pCR3.1-CERK1/pCR3.1) |
| C₆-ceramide | 1280 | 7 | 183 |
| Ceramide derived from bovine fetal brain | 200 | 10 | 20 |
| DG | 150 | 130 | 1 |
| D-erythro-sphingosine | 50 | 50 | 1 |

The protein of the present invention phosphorylated various ceramides such as C₆-ceramide and ceramide derived from bovine fetal brain, a natural type, with high efficiency. On the other hand, under the optimum assay conditions for the above-mentioned ceramide kinase, it hardly phosphorylated the lipids such as DG and sphingosine. Namely, it has high substrate specificity to ceramides.

### [Industrial applicability of the invention]

As described above, a novel protein having ceramide kinase activity and DNA encoding it are provided in the present invention. Furthermore, the present invention also provides a method for testing a ceramide kinase activator or inhibitor using the protein, and a specific and highly sensitive method for detecting or quantifying ceramide using the protein. The protein of the present invention or DNA encoding it are useful for searching for novel compounds which have a specifically activating or inhibiting activity against ceramide kinase as a medicament for treating neuronal disorder, an anti-inflammatory medicament, a medicament for treating HIV infection, an anti-type 2 diabetes mellitus medicament, an anti-obesity medicament, an anti-septicemia medicament, an anti-arteriosclerosis medicament and an anticancer medicament, and for analyzing the various conditions of the above-mentioned diseases.

## Claims

1. Aprotein having a property of the following i) or ii):
i) a protein which comprises an amino acid sequence shown in the amino acid numbers 1-537 of SEQ ID No. 2 of the Sequencing Listing;
ii) a protein which comprises an amino acid sequence wherein one or more than one amino acids are added, deleted and/or replaced in the amino acid sequence shown in the amino acid numbers 1-537 of SEQ ID No. 2 of the Sequencing Listing, and has ceramide kinase activity.

2. A protein comprising an amino acid sequence shown in the amino acid numbers 1-537 of SEQ ID No. 2 of the Sequencing Listing.

3. A protein encoded by DNA which is inserted in the plasmid carried by a transformed Escherichia coli E.coli pCR3.1-CERK1 SANK 70300 (FERM BP-7184).

4. A DNA which encodes a protein according to any one of Claims 1 to 3.

5. A DNA comprising a nucleotide sequence shown in the nucleotide numbers 124-1734 of SEQ ID No. 1 of the Sequencing Listing.

6. A DNA which hybridizes to a DNA comprising a nucleotide sequence shown in the nucleotide numbers 124-1734 of SEQ ID No. 1 of the Sequencing Listing under stringent conditions, and encodes a protein having ceramide kinase activity.

7. A DNA inserted in the plasmid which is carried by transformed Escherichia coli E.coli pCR3.1-CERK1 SANK 70300 (FERM BP-7184).

8. A recombinant DNA vector comprising DNA of any one of Claims 4 to 7.

9. A recombinant DNA vector according to claim 8, said recombinant DNA vector being carried by transformed Escherichia coli E.coli pCR3.1-CERK1 SANK70300 (FERM BP-7184).

10. A recombinant DNA vector according to claim 9, said recombinant DNA vector being an expression vector.

11. A host cell transformed with the recombinant DNA vector according to any one of Claims 8 to 10.

12. A host cell according to Claim 11, said host cell being transformed Escherichia coli E.coli pCR3.1-CERK1 SANK 70300 (FERM BP-7184).

13. A host cell transformed with the recombinant DNA vector according to Claim 10.

14. A method for producing a protein having ceramide kinase activity wherein a host cell according to Claim 13 is cultured under the conditions in which the protein having ceramide kinase activity can be produced, and subsequently the protein having ceramide kinase activity is collected from the culture.

15. A method for producing the protein according to any one of claims 1 to 5 comprising the following steps (i) to (iii):
(i) an extract of a material containing the protein according to any one of Claims 1 to 3 dissolved in a solvent which does not contain calmodulin is allowed to adsorb on affinity-chromatography resin having calmodulin as a substituent;
(ii) the resin of (i) is washed with a solvent which does not contain calmodulin;
(iii) the protein having ceramide kinase activity is eluted with a solvent containing EGTA or EDTA from the resin washed in the step (ii).

16. A method for testing the ceramide kinase activating or inhibiting effect of a compound or a composition sample, said method comprises the following steps (i) to (iv):
(i) preparing a mixture wherein a protein according to any one of claims 1 to 3, a substrate which can be phosphorylated specifically by the protein and said compound or composition sample are brought together, and a mixture wherein the protein and the substrate are bought together but the compound or composition sample is not added,
(ii) measuring and comparing the amount of the substrate phosphorylated in each of the mixtures prepared in (i).

17. A method according to claim 16, said method being **characterized in that** the substrate which can be phosphorylated specifically by a protein according to any one of claims 1 to 3 is one selected from the group consisting of C₂ ceramide, C₆ ceramide, C₈ ceramide, C₁₆ ceramide, and ceramide derived from bovine fetal brain.

18. A method for detecting or quantifying ceramide in a sample, said method being **characterized in that** a protein according to any one of claims 1 to 3, the sample to be tested, and labeled adenosine 5'-triphosphate are brought together, and then the labeled ceramide is detected or quantified.

19. Use of a protein according to any one of claims 1 to 3 for the production of a kit for detecting or quantifying ceramide.

20. An antibody which specifically binds to a protein according to any one of claims 1 to 3.

21. A pharmaceutical composition comprising a DNA according to any one of Claims 4 to 7 as an active ingredient.

22. A nucleic acid comprising the antisense sequence to a nucleotide sequence consisting of 15 to 30 continuous nucleotides in the nucleotide sequence shown in SEQ ID No.1 of Sequencing Listing.

23. A pharmaceutical composition comprising a nucleic acid according to claim 22.


SEQUENCE LISTING

<110> Sankyo Company, Limited

<120> Ceramide Kinase and DNA Encoding It

<130> EPP86264

<150> JP 2000-178039
<151> 2000-06-14

<160> 4

<170> PatentIn Ver. 2.0

<210> 1
<211> 4463
<212> DNA
<213> Homo sapiens

<220>
<221> CDS
<222> (124)..(1734)

<400> 1
gcgccgcctc ccgcagcccg ccgcccgccg cccgccgccc gggccactgc aggggccgct 60

aacggtccgg cgcccctcgg cgtccgcgcg cccccagcct ggcggacgag cccggcggcg 120

gag atg ggg gcg acg ggg gcg gcg gag ccg ctg caa tcc gtg ctg tgg 168
    Met Gly Ala Thr Gly Ala Ala Glu Pro Leu Gln Ser Val Leu Trp
      1               5                  10                  15

gtg aag cag cag cgc tgc gcc gtg agc ctg gag ccc gcg cgg gct ctg 216
Val Lys Gln Gln Arg Cys Ala Val Ser Leu Glu Pro Ala Arg Ala Leu
                 20                  25                  30

ctg cgc tgg tgg cgg agc ccg ggg ccc gga gcc ggc gcc ccc ggc gcg 264
Leu Arg Trp Trp Arg Ser Pro Gly Pro Gly Ala Gly Ala Pro Gly Ala
             35                  40                  45

gat gcc tgc tct gtg cct gta tct gag atc atc gcc gtt gag gaa aca 312
Asp Ala Cys Ser Val Pro Val Ser Glu Ile Ile Ala Val Glu Glu Thr
         50                  55                  60

gac gtt cac ggg aaa cat caa ggc agt gga aaa tgg cag aaa atg gaa 360
Asp Val His Gly Lys His Gln Gly Ser Gly Lys Trp Gln Lys Met Glu
    65                  70                  75

aag cct tac gct ttt aca gtt cac tgt gta aag aga gca cga cgg cac 408
Lys Pro Tyr Ala Phe Thr Val His Cys Val Lys Arg Ala Arg Arg His
80                  85                  90                  95

cgc tgg aag tgg gcg cag gtg act ttc tgg tgt cca gag gag cag ctg 456
Arg Trp Lys Trp Ala Gln Val Thr Phe Trp Cys Pro Glu Glu Gln Leu
                100                 105                 110

tgt cac ttg tgg ctg cag acc ctg cgg gag atg ctg gag aag ctg acg 504
Cys His Leu Trp Leu Gln Thr Leu Arg Glu Met Leu Glu Lys Leu Thr
            115                 120                 125

tcc aga cca aag cat tta ctg gta ttt atc aac ccg ttt gga gga aaa 552
Ser Arg Pro Lys His Leu Leu Val Phe Ile Asn Pro Phe Gly Gly Lys
        130                 135                 140

gga caa ggc aag cgg ata tat gaa aga aaa gtg gca cca ctg ttc acc 600
Gly Gln Gly Lys Arg Ile Tyr Glu Arg Lys Val Ala Pro Leu Phe Thr
    145                 150                 155

tta gcc tcc atc acc act gac atc atc gtt act gaa cat gct aat cag 648
Leu Ala Ser Ile Thr Thr Asp Ile Ile Val Thr Glu His Ala Asn Gln
160                 165                 170                 175

gcc aag gag act ctg tat gag att aac ata gac aaa tac gac ggc atc 696
Ala Lys Glu Thr Leu Tyr Glu Ile Asn Ile Asp Lys Tyr Asp Gly Ile
                180                 185                 190

gtc tgt gtc ggc gga gat ggt atg ttc agc gag gtg ctg cac ggt ctg 744
Val Cys Val Gly Gly Asp Gly Met Phe Ser Glu Val Leu His Gly Leu
            195                 200                 205

att ggg agg acg cag agg agc gcc ggg gtc gac cag aac cac ccc cgg 792
Ile Gly Arg Thr Gln Arg Ser Ala Gly Val Asp Gln Asn His Pro Arg
        210                 215                 220

gct gtg ctg gtc ccc agt agc ctc cgg att gga atc att ccc gca ggt 840
Ala Val Leu Val Pro Ser Ser Leu Arg Ile Gly Ile Ile Pro Ala Gly
    225                 230                 235

cca acg gac tgc gtg tgt tac tcc acc gtg ggc acc agc gac gca gaa 888
Pro Thr Asp Cys Val Cys Tyr Ser Thr Val Gly Thr Ser Asp Ala Glu
240                 245                 250                 255

acc tcg gcg ctg cat atc gtt gtt ggg gac tcg ctg gcc atg gat gtg 936
Thr Ser Ala Leu His Ile Val Val Gly Asp Ser Leu Ala Met Asp Val
                260                 265                 270

tcc tca gtc cac cac aac agc aca ctc ctt cgc tac tcc gtg tcc ctg 984
Ser Ser Val His His Asn Ser Thr Leu Leu Arg Tyr Ser Val Ser Leu
            275                 280                 285

ctg ggc tac ggc ttc tac ggg gac atc atc aag gac agt gag aag aaa 1032
Leu Gly Tyr Gly Phe Tyr Gly Asp Ile Ile Lys Asp Ser Glu Lys Lys
        290                 295                 300

cgg tgg ttg ggt ctt gcc aga tac gac ttt tca ggt tta aag acc ttc 1080
Arg Trp Leu Gly Leu Ala Arg Tyr Asp Phe Ser Gly Leu Lys Thr Phe
    305                 310                 315

ctc tcc cac cac tgc tat gaa ggg aca gtg tcc ttc ctc cct gca caa 1128
Leu Ser His His Cys Tyr Glu Gly Thr Val Ser Phe Leu Pro Ala Gln
320                 325                 330                 335

cac acg gtg gga tct cca agg gat agg aag ccc tgc cgg gca gga tgc 1176
His Thr Val Gly Ser Pro Arg Asp Arg Lys Pro Cys Arg Ala Gly Cys
                340                 345                 350

ttt gtt tgc agg caa agc aag cag cag ctg gag gag gag cag aag aaa 1224
Phe Val Cys Arg Gln Ser Lys Gln Gln Leu Glu Glu Glu Gln Lys Lys
            355                 360                 365

gca ctg tat ggt ttg gaa gct gcg gag gac gtg gag gag tgg caa gtc 1272
Ala Leu Tyr Gly Leu Glu Ala Ala Glu Asp Val Glu Glu Trp Gln Val
        370                 375                 380

gtc tgt ggg aag ttt ctg gcc atc aat gcc aca aac atg tcc tgt gct 1320
Val Cys Gly Lys Phe Leu Ala Ile Asn Ala Thr Asn Met Ser Cys Ala
    385                 390                 395

tgt cgc cgg agc ccc agg ggc ctc tcc ccg gct gcc cac ttg gga gac 1368
Cys Arg Arg Ser Pro Arg Gly Leu Ser Pro Ala Ala His Leu Gly Asp
400                 405                 410                 415

ggg tct tct gac ctc atc ctc atc cgg aaa tgc tcc aag ttc aat ttt 1416
Gly Ser Ser Asp Leu Ile Leu Ile Arg Lys Cys Ser Lys Phe Asn Phe
                420                 425                 430

ctg aga ttt ctc atc agg cac acc aac cag cag gac cag ttt gac ttc 1464
Leu Arg Phe Leu Ile Arg His Thr Asn Gln Gln Asp Gln Phe Asp Phe
            435                 440                 445

act ttt gtt gaa gtt tat cgc gtc aag aaa ttc cag ttt acg tcg aag 1512
Thr Phe Val Glu Val Tyr Arg Val Lys Lys Phe Gln Phe Thr Ser Lys
        450                 455                 460

cac atg gag gat gag gac agc gac ctc aag gag ggg ggg aag aag cgc 1560
His Met Glu Asp Glu Asp Ser Asp Leu Lys Glu Gly Gly Lys Lys Arg
    465                 470                 475

ttt ggg cac att tgc agc agc cac ccc tcc tgc tgc tgc acc gtc tcc 1608
Phe Gly His Ile Cys Ser Ser His Pro Ser Cys Cys Cys Thr Val Ser
480                 485                 490                 495

aac agc tcc tgg aac tgc gat ggg gag gtc ctg cac agc cct gcc atc 1656
Asn Ser Ser Trp Asn Cys Asp Gly Glu Val Leu His Ser Pro Ala Ile
                500                 505                 510

gag gtc aga gtc cac tgc cag ctg gtt cga ctc ttt gca cga gga att 1704
Glu Val Arg Val His Cys Gln Leu Val Arg Leu Phe Ala Arg Gly Ile
            515                 520                 525

gaa gag aat ccg aag cca gac tca cac agc tgagaagccg gcgtcctgct 1754
Glu Glu Asn Pro Lys Pro Asp Ser His Ser
        530                 535

cacaaactgg gaaagtgtga aaactattta agataattat tacagaccaa ttatgttgat 1814

atatacattt aaatgtagaa atttattttt gatagttaaa tcttgatttt agaagaaaac 1874

ccttttgtca acaattttgt gtacatattt ggcattttca gttctgtacg catctgcggg 1934

ttgcagccca cgccgcttac tctcagcgga tgcagctgct cacttggggg cactggcctc 1994

ttaggtttta acgatgtcaa cagtgtagtt tagaaaatgg cccgttagtg gctctattgc 2054

aataatgtta gggacattat atgatttcca cgcaggtcac accatctggg cctgaggtag 2114

cagtgggtca ctttgatcca ctttgcagga cttattctgt aacggtttgt ggccaagttt 2174

tgggaagtgg ttgattatct ttgccttcat ttcaccttcc tcttcgttta cggttaggac 2234

atcgctgctt gatccttaca atactgtgca actgcaatgc aacgtggccc tgcttcaggt 2294

gatccgcggg aggggcctcc acgccagcgc cgggaaggct gctggggcct ccacacctgc 2354

ctcatcacgg cggcgaggct acgacaatcc ggctgggagc atgaccttgg cgtctgttct 2414

gggagcacag atgataagct ctggaagctg gcagtgtgta aagcactggc aagtttgtta 2474

ctgttaaaat gtcaaatacc aatgctttat atcgacgcga agtgcttaac acagccgggc 2534

ttgggggcag tcaggaggaa gctggccatc cgtggaggag gggccggtcc tggactcccg 2594

caggactcct ctgatgcagg gcctgaagtc tgtacacgtg gtccaaattt gtccttgtct 2654

tttcttcaca ctgagttctc tatatttatt gaacatcttg tccttttaag ccagagtagt 2714

gtaaactgcg tctcggatgt ctgtcttttg tgtcgaagcc acgatggatc gctggtttcc 2774

tctgcagcgc aagggctccg gcgaccagag gattcttccc ggaaaggcat tcctgccgcg 2834

ctccccgggg cacccctcaa ttgtgtacta cgtccttgtt tagtgtgtat ccgtgcccac 2894

gtagatgatg tctgtaacgt agttttgttt gaaatatgag aatatgcggc ttaaactttg 2954

atctgtaagg agcggggccg tggccgtttg gagcacgctg tagacaccgt tcctcatgct 3014

gccgggtggg ttttgcagaa gctcccttag tgatttcatg tttaacaggc agcatccatt 3074

ttcagaattt cctggcattg atttatattt tgaagcatac aggaaacttc tcgtttcctc 3134

gtttagcccc acccagatca ggtgaaaggg cagctttaat ggtggttttt atggaccaca 3194

ttatcagaga gcactgtgca agccaaatgg ttcaataatg aatgaaaatt ctgggtgtaa 3254

agagtaaata tgccctggct ctttctacca atgtttgctc ctggttggaa agaaaccaaa 3314

gatttaagac gggctgctct tccagactgg ctgtgcctgc ctgtgcccag caacctntgc 3374

agccggcagt gtgcctggtg tcacgccagg aggctgtggc tgctgtgggc cctctggaat 3434

tgtgctcctc acaaagtttc cccaaaaggt tcttctaagc ctttattgtc cctggtaaat 3494

gtttcccggc tgggcgcggt ggctcacgcc tgtaatccca gcactttggg aggccgaggc 3554

gggtggatca cctaaggtca ggagtttgag atcagcctgc ccaacatggt gaaacctcgt 3614

ctctactaaa aatacacaac ttagccagtc ttgttggcgc acgcctgtaa tctcagctac 3674

tagggatgct gaggcaggag aatcgcttga acccaagaaa gaggtggagg ttgcggtgag 3734

ccaagattgc gccactgcac tccagcctgg gcaacagagg gagactccat cgccccccct 3794

caacaacaaa aaaagtttcc catacactgg cctgccccaa aacccactaa caattttagc 3854

aaaacagtcc agccaaagag gaagcatttc atgttcaata agaaacccag ccattccgca 3914

tggctggttc ctgagtggct ctggtgatac tctccagcca cctgctgaca ttcagaatct 3974

cagacctcgg ggactgctgt tgcggtaccg tgtgtctgac acctgccagc agccctttgc 4034

tatctgcgcg caggatgggg gtgactgccc agacattccc gctagatagg ctctgatttc 4094

cggggcagcc tttcagatgc ggcagacata caacacctgt actttagagt tttaagggaa 4154

aaaaaaatca gaagtgctgg ttagatagta aaaacttagg ataacttaga aaggctagtt 4214

ttagcttcct ttgtggctcc ctggtgcaaa acaattagca gttatgcaat ggacctgatt 4274

ctagtttatt ctaattaaga agtgaggccg ggtttgactt cgttcctgaa tacaatcttg 4334

agtaactggg aaagtctgag tgaaaggatg gcctcattct ctttctaatc ttgctggttt 4394

caagattaga aaatggcatt atttgatctg aaatgtttga gaagacacga ataaagctcg 4454

tgccgaatt 4463

<210> 2
<211> 537
<212> PRT
<213> Homo sapiens

<400> 2

Met Gly Ala Thr Gly Ala Ala Glu Pro Leu Gln Ser Val Leu Trp Val
1               5                   10                  15

Lys Gln Gln Arg Cys Ala Val Ser Leu Glu Pro Ala Arg Ala Leu Leu
            20                  25                  30

Arg Trp Trp Arg Ser Pro Gly Pro Gly Ala Gly Ala Pro Gly Ala Asp
        35                  40                  45

Ala Cys Ser Val Pro Val Ser Glu Ile Ile Ala Val Glu Glu Thr Asp
    50                  55                  60

Val His Gly Lys His Gln Gly Ser Gly Lys Trp Gln Lys Met Glu Lys
65                  70                  75                  80

Pro Tyr Ala Phe Thr Val His Cys Val Lys Arg Ala Arg Arg His Arg
                85                  90                  95

Trp Lys Trp Ala Gln Val Thr Phe Trp Cys Pro Glu Glu Gln Leu Cys
            100                 105                 110

His Leu Trp Leu Gln Thr Leu Arg Glu Met Leu Glu Lys Leu Thr Ser
        115                 120                 125

Arg Pro Lys His Leu Leu Val Phe Ile Asn Pro Phe Gly Gly Lys Gly
    130                 135                 140

Gln Gly Lys Arg Ile Tyr Glu Arg Lys Val Ala Pro Leu Phe Thr Leu
145                 150                 155                 160

Ala Ser Ile Thr Thr Asp Ile Ile Val Thr Glu His Ala Asn Gln Ala
                165                 170                 175

Lys Glu Thr Leu Tyr Glu Ile Asn Ile Asp Lys Tyr Asp Gly Ile Val
            180                 185                 190

Cys Val Gly Gly Asp Gly Met Phe Ser Glu Val Leu His Gly Leu Ile
        195                 200                 205

Gly Arg Thr Gln Arg Ser Ala Gly Val Asp Gln Asn His Pro Arg Ala
    210                 215                 220

Val Leu Val Pro Ser Ser Leu Arg Ile Gly Ile Ile Pro Ala Gly Pro
225                 230                 235                 240

Thr Asp Cys Val Cys Tyr Ser Thr Val Gly Thr Ser Asp Ala Glu Thr
                245                 250                 255

Ser Ala Leu His Ile Val Val Gly Asp Ser Leu Ala Met Asp Val Ser
            260                 265                 270

Ser Val His His Asn Ser Thr Leu Leu Arg Tyr Ser Val Ser Leu Leu
        275                 280                 285

Gly Tyr Gly Phe Tyr Gly Asp Ile Ile Lys Asp Ser Glu Lys Lys Arg
    290                 295                 300

Trp Leu Gly Leu Ala Arg Tyr Asp Phe Ser Gly Leu Lys Thr Phe Leu
305                 310                 315                 320

Ser His His Cys Tyr Glu Gly Thr Val Ser Phe Leu Pro Ala Gln His
                325                 330                 335

Thr Val Gly Ser Pro Arg Asp Arg Lys Pro Cys Arg Ala Gly Cys Phe
            340                 345                 350

Val Cys Arg Gln Ser Lys Gln Gln Leu Glu Glu Glu Gln Lys Lys Ala
        355                 360                 365

Leu Tyr Gly Leu Glu Ala Ala Glu Asp Val Glu Glu Trp Gln Val Val
    370                 375                 380

Cys Gly Lys Phe Leu Ala Ile Asn Ala Thr Asn Met Ser Cys Ala Cys
385                 390                 395                 400

Arg Arg Ser Pro Arg Gly Leu Ser Pro Ala Ala His Leu Gly Asp Gly
                405                 410                 415

Ser Ser Asp Leu Ile Leu Ile Arg Lys Cys Ser Lys Phe Asn Phe Leu
            420                 425                 430

Arg Phe Leu Ile Arg His Thr Asn Gln Gln Asp Gln Phe Asp Phe Thr
        435                 440                 445

Phe Val Glu Val Tyr Arg Val Lys Lys Phe Gln Phe Thr Ser Lys His
    450                 455                 460

Met Glu Asp Glu Asp Ser Asp Leu Lys Glu Gly Gly Lys Lys Arg Phe

465                 470                 475                 480

Gly His Ile Cys Ser Ser His Pro Ser Cys Cys Cys Thr Val Ser Asn
                485                 490                 495

Ser Ser Trp Asn Cys Asp Gly Glu Val Leu His Ser Pro Ala Ile Glu
            500                 505                 510

Val Arg Val His Cys Gln Leu Val Arg Leu Phe Ala Arg Gly Ile Glu
        515                 520                 525

Glu Asn Pro Lys Pro Asp Ser His Ser
    530                 535

<210> 3
<211> 33
<212> DNA
<213> Artificial Sequence

<220>
<223> Description of Artificial Sequence: PCR primer to
      amplify a fragment of cDNA encoding human ceramide
      kinase

<400> 3
tcaccactga catcatcgtt actgaacatg cta                              33

<210> 4
<211> 35
<212> DNA
<213> Artificial Sequence

<220>
<223> Description of Artificial Sequence: PCR primer to
      amplify a fragment of cDNA encoding human ceramide
      kinase

<400> 4
caacgatatg cagcgccgag gtttctgcgt cgctg                            35